# EUROPÄISCHE PATENTSCHRIFT

(11) **EP 0 759 900 B1**
(45) Veröffentlichungstag und Bekanntmachung des Hinweises auf die Patenterteilung: **24.06.1998**
(21) Anmeldenummer: 95917278.4
(22) Anmeldetag: 03.05.1995
(51) Int. Cl.: C07C 209/84

(54) **VERFAHREN UND VORRICHTUNG ZUR RÜCKGEWINNUNG VON AMINEN UND VERWENDUNG VERFAHRENSGEMÄSS ERHALTBARER RÜCKSTÄNDE**
PROCESS AND DEVICE FOR RECOVERING AMINES AND USE OF RESIDUES OBTAINABLE THEREBY
PROCEDE ET DISPOSITIF DE RECUPERATION D'AMINES ET UTILISATION DES RESIDUS AINSI OBTENUS

(30) Priorität: 11.05.1994 DE 4416571
(43) Veröffentlichungstag der Anmeldung: 05.03.1997
(73) Patentinhaber: Giebeler, Eberhard, 39218 Schönebeck/Elbe (DE)
(72) Erfinder: Giebeler, Eberhard, 39218 Schönebeck/Elbe (DE)
(86) Internationale Anmeldenummer: DE9500595
(87) Internationale Veröffentlichungsnummer: WO9531430

(56) Entgegenhaltungen:
- EP-A- 0 267 531
- EP-A- 0 494 543
- DE-A- 3 104 343
- US-A- 4 472 246

## Beschreibung

Amine werden in zahlreichen Prozessen als Reaktionspartner, Lösungsmittel und Katalysatoren eingesetzt. Beispiele sind die Herstellung von quaternären Ammoniumverbindungen und die katalytische Aushärtung von Polyol-Isocyanat-Bindemittelsystemen (z.B.Coldbox-Verfahren zur Härtung von Giessereikernsanden).

Wegen des unangenehmen Geruchs und der niedrigen Toxizitätsschwelle der Amine muß die Abluft bei derartigen Prozessen nachbehandelt werden, um die Amine entweder sicher zu entsorgen oder zurückzugewinnen.

Ein verbreitetes Gasreinigungsverfahren ist die thermische Nachverbrennung, bei der die Amine verbrannt werden. Bei den Nachverbrennungen ist es durch aufwendige Reaktionsführung und Ammoniakzugabe möglich, die Stickoxidemission zu beherrschen. Dieses Verfahren ist aber in kleineren Anlagen mit diskontinuierlichem Betrieb wie z.B. in Coldbox-Kernherstellanlagen in Gießereibetrieben in der Regel nicht wirkungsvoll zu kontrollieren. Nachteilig ist in jedem Fall, daß dabei eine wertvolle Chemikalie verbrannt wird, die zudem in einer für eine Verbrennung ungünstigen hohen Verdünnung vorliegt.

Ein anderes verbreitetes Verfahren ist die chemische Absorption der in der Abluft enthaltenen Amine in Mineralsäuren. Als Absorber dienen z.B. Sprühabsorber oder Füllkörperkolonnnen mit Gegenstromführung. In einer 15-40 %igen Schwefelsäure werden je nach Amin und Säurekonzentration in der Praxis Aminbeladungen im Bereich 10-30 % erzielt. Wesentlich ist, daß in einem pH-Bereich unter pH 3 gearbeitet wird, um Aminemissionen zu vermeiden. Der Vorteil des Verfahrens ist, daß die Amine verfahrenstechnisch einfach und sicher in der Säure konzentriert werden.

Anstelle von Schwefelsäure können auch andere Säuren, z.B. Phosphorsäure. eingesetzt werden. Die preiswerte Schwefelsäure ist aber bevorzugt.

Durch die Absorption ist das Entsorgungs- und Umweltproblem primär aus der Abluft in die mit Amin beladene Säure verlagert worden. Das Folgeproblem ist die Entsorgung und Verwertung der gesättigten Waschlösungen. In der Praxis werden folgende Entsorgungswege für die erschöpfte Absorberflüssigkeit beschritten:

### 1. Verdünnung mit dem Abwasser nach Neutralisation und Abbau der Amine in einer biologischen Kläranlage.

Damit sind die Amine ohne Nutzung verloren. Die hohe Salzfracht bedeutet einen Beitrag zur Aufsalzung der Gewässer und stellt im Fall der üblichen Anwendung von Schwefelsäure als Absorptionsflüssigkeit eine bedeutende Korrosionsquelle für Abwasserleitungen aus Beton dar. Außerdem entweicht nach dem erforderlichen Neutralisieren des Abwassers ein Teil der Amine als gasförmige Emission.

### 2. Verbrennung der Aminsulfatlösungen in Spaltöfen.

Dabei werden die Amine verbrannt und die Sulfate werden zu Schwefeloxiden umgesetzt. In besonders für diesen Zweck konstruierten Anlagen kann das Schwefeldioxid z.B. zur Schwefelsäureherstellung genutzt werden. Dises Verfahren ist aufwendig und bedeutet einen Verlust der Amine, die wertvolle Chemikalien sind.

### 3. Rückgewinnung der Amine aus den Waschlösungen

In der Patentschrift DE 31 04 343 A1 (Arasin) wird ein Aminrecyclingverfahren beschrieben, das von Phosphorsäure als Absorptionsflüssigkeit ausgeht. Die beladene Aminphosphatlösung wird in der Recyclinganlage mit Calciumoxid oder Calciumhydroxid neutralisiert. Unter Rühren und Erwärmen bis max. 105 °C werden die Amine in einem Rührkessel freigesetzt und anschließend kondensiert. Zur Entwässerung der anfallenden Amin/Wasser-Azeotrope werden Molekularsiebe vorgeschlagen.

Die Patentschrift nimmt auch Stellung zu der Aufarbeitung des aus einer Suspension von Calciumhydroxid und Calciumphosphat bestehenden Sumpfes, der im Rührkessel zurückbleibt. Dieser wird mit Phosphorsäure neutralisiert. Anschließend läßt man den Feststoff absetzen und filtriert den abgesetzten Schlamm. Die überstehende Lösung ist erfahrungsgemäß wegen des hohen Feststoffgehalts nicht vollständig deaminiert; dies ist der Grund, weshalb der Wiedereinsatz dieser Lösung in der Deaminierungsstufe beschrieben ist.

Zur Trocknung des - wie die überstehende Lösung - ebenfalls noch aminhaltigen Filterkuchens werden bekannte Trocknerbauarten wie Wirbelbett- und Hordentrockner genannt. Der getrocknete Filterkuchen besteht aus Calciumphosphat, einem verwertbaren Rohstoff. Der Prozeß ist durch seine vielen Bearbeitungsstufen und den Einsatz der Phosphorsäure teuer und aufwendig. In der Verfahrensbeschreibung bleibt die wesentliche Frage, wie die problematischen Aminemissionen bei dem vielstufigen Handling des Feststoffs vermieden werden, unbeantwortet. Dies ist vermutlich - neben dem hohen Aufwand für das Verfahren - ein Grund dafür, daß dieses Verfahren in der Praxis nicht angewandt wird. Es wird auch kein gangbarer Weg zur Entwässerung der Amin/Wasser-Azeotrope aufgezeigt, insbesondere die Art und Wirkung des Molsiebs und dessen Desorption bleiben offen.

Die Patentschrift US 4.472.246 (Ashland Oil 1984) beschreibt ein Verfahren zur Rückgewinnung der Amine, das in der Praxis angewendet wird. Dabei wird die ursprünglich saure Waschlösung mit anorganischen Basen aus der Reihe der Alkali- und Erdalkalimetallhydroxide vermischt und über den Siedepunkt der Amine enwärmt. Während das Verfahren für Amine, die keine Azeotrope mit Wasser bilden, gut anwendbar scheint, ist die beschriebene Trennung von Amin/Wasser-Azeotropen durch Phasentrennungen und Redestillationen sehr aufwendig und vermutlich in der Praxis kaum durchführbar. In den Patentansprüchen und im Beisspiel fällt die ausdrückliche Beschränkung auf Amine, die keine Azeotrope mit Wasser bilden, wie Dimethylethylamin, auf.

Mit der Verwertung des Koppelprodukts, dem Salzrückstand bzw der Salzlösung im Blasensumpf der Destillation, beschäftigt sich das oben beschriebene Patent nicht. Dieses Problem ist mit dem Entfernen der Salzlösung aus der Blase nicht gelöst und stellt den Schwachpunkt des oben beschriebenen Verfahrens dar.

Das im Patent der Ashland Oil in einem Beispiel beschriebene und heute in der Praxis übliche Verfahren verwendet 50%ige Natronlauge als Base. Natronlauge bietet neben dem günstigen Preis und der guten Verfügbarkeit den wesentlichen Vorteil, daß die Löslichkeit des bei der Umsetzung mit Aminsulfat gebildeten Natriumsulfats bei hohen Temperaturen gut ist, z.B. im Vergleich zu Kaliumsulfat oder Calciumsulfat.

| Löslichkeiten von Salzen in Wasser bei 100 C: | |
|---|---|
| Natriumsulfat | 43 g/100 ml Wasser |
| Kaliumsulfat | 24 g/100 ml Wasser |
| Calciumsulfat | 0,16 g/100 ml Wasser |

Diese hohe Löslichkeit des Natriumsulfats bietet den Vorteil, daß in der Destillationsblase Konzentrationsniederschläge vermieden werden. Eine Verkrustungsgefahr der Wärmeaustauscherflächen mit Natriumsulfat ist dennoch in gewissem Umfang gegeben.

Im Falle der Verwendung von Kalilauge müßte mit annähernd der doppelten Verdünnung wie bei Natronlauge gearbeitet werden, um Konzentrationsniederschläge beherrschen zu können. Im Falle des Einsatzes von Kalk als billigster Base würde in jedem Fall ein Niederschlag von Calciumsulfat entstehen. In diesem Schlamm werden Aminsulfate und Amine eingeschlossen, und eine vollständige destillative Abtrennung der Amine wäre auch wegen des schlechten Wärmeübergangs in dem Schlamm erschwert oder gar in herkömmlichen Destillationsblasen unmöglich.

Die Verwendung von Calciumhydroxid, obwohl in den Patentansprüchen der Ashland Oil mit erfaßt, ist in der Praxis bei der Deaminierung der Aminsulfatlösungen in üblichen Destillationsblase aus den obengenannten Gründen praktisch nicht durchführbar und wird deshalb nicht angewandt, obwohl Kalkprodukte die billigsten Basen sind.

In der Praxis ist die Verwendung von Natronlauge zur Aminrückgewinnung aus den oben beschriebenen Gründen die angewandte Lösung, obwohl die anschließende Entsorgung des wertlosen Destillationssumpfes, einem Gemisch aus Natronlauge, Wasser und Natriumsulfat, bei steigenden ökologischen Anforderungen ein gravierendes Problem darstellt.

Insgesamt ist die fehlende oder unzureichende Verwertbarkeit dieser nach der Aminabtrennung noch mit prozeßtypischen Produkten kontaminierten Rückstände der wesentliche Schwachpunkt des Aminrecyclingverfahrens. Im Fall des Coldbox-Verfahrens (Aushärtung von Gießereisandbindemitteln auf Polyol/Isocyanatbasis mit aminischen Katalysatoren) werden z.B. in die saure Waschlösung feinste Quarzteilchen und organische Lösungsmittel eingetragen. Diese Kontaminationen tragen mit dazu bei, daß die Entsorgung oder Verwertung der salzhaltigen Rückstände ein Problem darstellt. Die Menge der zu entsorgenden Rückstände ist annähernd so groß wie die ursprünglich eingesetzte Aminsulfatmenge, d.h. durch das Recyclingverfahren tritt keine Mengenentlastung bei der Reststoffentsorgung ein.

Folgende Entsorgungswege für den Destillationsrückstand bieten sich an:
1. Neutralisation des alkalischen Sumpfes und Kristallisation des Natriumsulfats. Dieses Verfahren ist aufwendig und führt wegen prozeßbedingter Kontaminationen des Natriumsulfats in der Regel nicht zu einem verwertbaren Natriumsulfat. Natriumsulfat als unerwünschtes Nebenprodukt stellt ein bekanntes Verwertungsproblem dar, so daß die Deponierung des Natriumsulfats ein Ausweg ist. Die in der Regel anhaftenden minimalen Aminmengen stören aber geruchlich so, daß dieser Weg keine Praxisbedeutung hat.
2. Dosierung der heißen Natriumsulfatlösung aus isolierten, beheizbaren Tanks in das Abwassernetz einer Kläranlage. Dies ist die bestehende Praxis. Die unerwünschte Folge ist wiederum ein Aufsalzeffekt des Abwassers und die Betonkorrosionsgefahr durch die Sulfationen.

Zusammengefaßt ergibt sich, daß das in der Patentschrift der Ashland Oil beschriebene Verfahren nur dann sinnvoll und handhabbar ist, wenn der Destillationssumpf ohne Gefahr der Betonkorrosion in das Abwasser geleitet werden kann. Von dieser Situation geht die Patentschrift auch offensichtlich aus. Dies ist aber aus Korrosions- und Gewässerschutzgründen nicht immer erlaubt und zulässig. Ein weiterer Nachteil des von Ashland Oil beschriebenen Verfahrens ist die praktische Beschränkung auf Amine, die keine Azeotrope mit Wasser bilden. Amine, die Azeotrope mit Wasser bilden, wie z.B. Triethylamin mit 10 % Wasser im Azeotrop haben eine erhebliche Marktbedeutung.

Es ist daher die Hauptaufgabe der Erfindung, ein einfaches Verfahren zum Recycling von allen Aminen, insbesondere allen alifatischen Aminen, bereit zu stellen, das eine Verwertungsmöglichkeit für alle Nebenprodukte liefert, das die mit der Destillation und dem Handling von Schlämmen und übersättigten Salzlösungen verbundenen Probleme vermeidet und zu einem gut handhabbaren Feststoff als Destillationsrückstand führt.

Die Lösung vorstehender Aufgaben erfolgt durch die im Kennzeichen des Anspruchs 1 enthaltenen Merkmale. Weitere vorteilhafte Merkmale sind in den Unteransprüchen 2-15 genannt. In den Ansprüchen 10-12 ist eine spezielle Vorrichtung beschrieben, die außer für das Aminrecycling und die Entwässerung von Amin/Wasser-Azoetropen auch allgemein für die Trennung von Schlämmen in einen Feststoff und mehrere Fraktionen von verdampfbaren Flüssigphasenkomponenten vorteilhaft genutzt werden kann.

Es wurde überraschenderweise gefunden, daß insbesondere ein aus Calciumoxid oder Calciumhydroxid und Aminsulfatlösung erzeugter Schlamm handhabbar ist und vollkommen deaminiert werden kann, wenn die Neutralisation mit nachfolgender Deaminierung und thermischer Trocknung des Destillationssumpfs in einem speziellen Knettrockner durchgeführt wird, wobei das Calciumoxid oder Calciumhydroxid in einem stöchiometrischen Überschuß zum Sulfatgehalt der Waschlösung eingesetzt wird. Aus wirtschaftlichen Gründen wird man normalerweise nicht über das 1,5fache des stöchiometrischen Überschusses gehen. Die untere Grenze liegt in der Praxis bei einem 5 - 15 % igen stöchiometrischen Überschuß. Gebrannter oder gelöschter Kalk kann auch partiell durch Calciumcarbonat zur Neutralisation substituiert werden.
Ein besonderer Vorteil des Knettrockners und insbesondere der in Anspruch 10 beschriebenen Vorrichtung ist, daß damit auch in der gleichen Anlage ein einfaches Verfahren zur Entwässerung von Amin/Wasser-Azeotropen und zur Reindarstellung dieser Amine möglich ist.

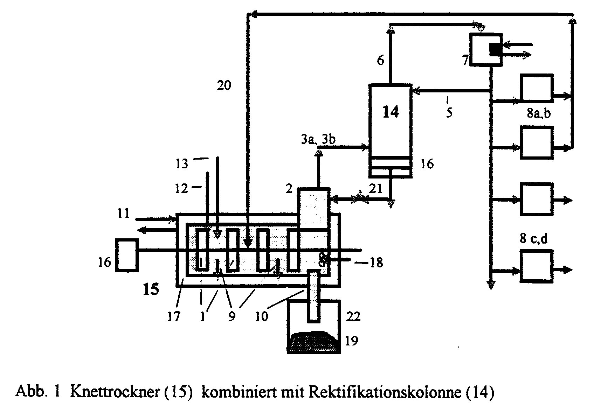

Bevorzugte Ausführungsformen des Knettrockners (15) in Abb. 1 weisen neben einer Wandheizung (17) auch mit Thermalöl oder Wasserdampf (11) beheizte Knet- und Mischwerkzeuge (1) auf, deren konstruktive Gestaltung eine intensive Oberflächenerneuerung und Durchmischung der in der Endphase der Trocknung entstehenden Zähphase ermöglicht. Kennzeichen dieser Knettrockner sind enge Spalte zwischen bewegten und statischen Knetorganen im Bereich 1,5 - 7 mm. Zur Verbesserung des Aufschlusses und zur Herstellung einer kleinen mittleren Korngröße können in den von den Knetorganen nicht bestrichenen Zonen rotierende Schneidmesser (18) angebracht werden. Derartige Trockner sind selbstreinigend; temporäre Verkrustungen an den Heizflächen werden immer wieder abgebaut. Als Beispiel für einen Knettrockner sind insbesondere die Knettrockner der Baureihe Discotherm der Fa. List AG, Schweiz zu enwähnen, die in der Lage sind sehr zähe Phasen im Endstadium der Trocknung zu brechen. Der Antriebsmotor (16) ist entsprechend stark ausgelegt.

Anstelle der indirekten Heizung ist auch eine direkte Heizung z.B. mit Wasserdampf möglich, wegen der damit verbundenen Erhöhung der Wassermenge im System aber nicht bevorzugt.

Der Trocknungsprozeß erfolgt durch Verdampfen des aus der Umsetzung der Aminsalzlösung (13) z.B. mit dem Calciumoxid (12) freigesetzten Amins (3a) und annähernd der gesamten Wassermenge (3b), bis auf einen Restwassergehalt von 0,5 - ca. 20 %. Ein wesentlich höherer Wassergehalt führt nicht zu rieselfähigen Feststoffen, ist aber gundsätzlich ebenfalls möglich, wenn die rieselfähige Konsistenz nicht gefordert ist. Da die Endtrocknung bei Normaldruck oder einem leichten Überdruck vorzugsweise bei Materialtemperaturen zwischen 120 °C und 170 °C oder bei Vakuumtrocknung im Bereich 100 - 150 °C erfolgt, werden in diesem Endstadium neben Restmengen von Aminen auch höhersiedende organische Kontaminationen wie z.B. Kohlenwasserstoffe, die im Coldbox-Verfahren als Lösungsmittel eingesetzt werden, zusammen mit dem Wasserdampf praktisch vollständig vom anorganischen Sumpf abgetrennt.

Nach diesem Verfahren kann in einem einzigen Arbeitsschritt neben dem Amin ein vollkommen deaminierter rieselfähiger Destillationsrückstand (19) gewonnen werden, der über eine geeignete Schleuse, z.B. eine Zellradsehleuse (10), in eine Vorlage (22) ausgetragen wird. Die Korngrößenverteilung kann durch die Einstellung des Trocknungsgrades, die Dimensionierung der Mischwerkzeuge und die Rotationsgeschwindigkeit des Kneters und das Temperaturprogramm beeinflußt und gesteuert werden.

Im Falle der Verwendung von Kalk und Kalkprodukten wie gelöschtem oder gebranntem Kalk als Basen ist ohne vorhergehende Neutralisation des Rückstands eine Verwertung des festen Rückstands bei der Zementherstellung gegeben, da die Inhaltsstoffe Calciumsulfat und Calciumoxid bzw. Calziumhydroxid sowie Quarz Rohstoffe für die Zementherstellung sind.

Wenn die Deaminierung bewußt bei einem möglichst niedrigen stöchiometrischen Calciumüberschuß durchgeführt wird, entsteht als Rückstand ein relativ reiner Gips mit geringem Calciumoxidüberschuß. Bei der Trocknung bei Temperaturen über 120 °C bildet sich bei Normaldruck bevorzugt die Gipsmodifikation des Beta-Halbhydrats, die auch direkt in der Gipsindustrie als Baustoff eingesetzt werden kann. Wenn nach der Deaminierung des Gipsschlamms die Trocknung unter einem Überdruck von bevorzugt 3-5 bar durchgeführt wird bei Temperaturen bis 160 °C , bildet sich unter diesen hydrothermalen Bedingungen bekanntlich bevorzugt die Modifikation des Alpha-Halbhydrats, die als Gipsbaustoff mit besonderen Fließeigenschaften in hochwertigen Anwendungen eingesetzt wird. Es kann erforderlich sein, den angewandten Calciumüberschuß durch Zugabe von Schwefelsäure zu reduzieren, um einen möglichst reinen Gips zu erzeugen, der für die Kristallbildung erforderlich ist. Der Vorteil der Erzeugung dieser hochwertigen Gipsmodifikation während des Prozesses der Deaminierung liegt darin, daß der Knettrockner diese Variante ohne weitere Installationen und wesentliche Kosten ermöglicht. Es ist alternativ zur Herstellung der Halbhydrate auch möglich, die Restentwässerung bei Temperaturen unter 50 °C im Vakuum oder durch einen Luftstrom durchzuführen, um gezielt Gips als Dihydrat herzustellen. In diesem Fall wird die Abluft in einen Aminwäscher geleitet, um Restmengen von Aminen auszuwaschen.

Es ist den jeweiligen Marktbedingungen überlassen, welche Gipsmodifikation zu bevorzugen ist.

In einer ebenfalls moglichen Variante des Verfahrens wird anstelle von Kalk oder Kalkprodukten Kalilauge zur Neutralisation eingesetzt. Der, wie oben beschrieben, in einer einzigen kompakten Anlage mit diskontinuierlicher Destillation/Trocknung gewonnene feste Rückstand Kaliumsulfat oder Kaliumphosphat kann nach Neutralisation als Düngemittelkomponente verwertet werden.

Falls andere Basen als Kalk/Kalkprodukte oder Kaliumhydroxid zur Neutralisation eingesetzt werden, wird ebenfalls der Vorteil eines konzentrierten rieselfähigen Rückstands erreicht, aber es ergeben sich keine günstigen Verwertungsmöglichkeiten für diesen Feststoff.

Die wirtschaftlich bevorzugte Variante ist die Verwendung von Calciumoxid oder Calciumhydroxid als Base und die Verwertung des Gipses in der Gipsindustrie. Calciumoxid hat neben dem niedrigen Preis den Vorteil, daß die Hydratation und Neutralisation stark exotherm verlaufen und diese Reaktionswärme für die Destillation genutzt werden kann. Diese Exothermie kann anderseits durch Zumischung von Calciumcarbonat als Base reduziert werden

### Beispiel 1

In einem 7 l-Knettrockner mit beheizten Knetscheiben (Typ und Hersteller: LIST AG DTB Batch) und einer Heizfläche von 0,3 m² wurden 3000 g Calciumhydroxid (gelöschter Kalk) vorgelegt. Anschließend wurden bei Raumtemperatur und Normaldruck 5566 g einer Aminsulfatlösung mit der Zusammensetzung:
18 % Schwefelsäure
70 % Wasser
12 % Amin (Gemisch aus Dimethylethylamin DMEA (84 %), Dimethylisopropylamin DMIPA (6 %) und Triethylamin TEA (8 %)

Der pH-Wert der sich bildenden Suspension aus Kalk und Calciumsulfat lag bei 12. Die Reaktionswärme führte zu einer meßbaren Temperaturerhöhung von 24 °C auf 27 °C. Die Thermalöltemperatur für die indirekte Beheizung des Trockners wurde auf 100 C eingestellt. Ab 55 °C in der Suspension wurde eine merkliche Verdampfung und Kondensation der Amine bei Normaldruck festgestellt. 700 ml organische Phase wurden bei einer Blasentemperatur (Temperatur der Suspension im Knettrockner) bis 77 °C verdampft. Die Anhebung der Thermalöltemperatur auf 150 °C, dann 200 °C führte zu einer raschen Verdampfung der restlichen Amine und des Wassers.
Insgesamt wurden
1030 ml als Aminfraktion (Sdp. <100 °C), dann
1400 ml einer wässrigen Phase mit leichtem Amingeruch, dann
3400 ml aminfreies Wasser abdestilliert.

Bei Normaldruck wurde bis zu einer Blasentemperatur von 170 °C Wasser abdestilliert. 2 h nach Versuchsbeginn war die diskontinuierliche Destillation/Trocknung beendet.

| | | | | | |
|---|---|---|---|---|---|
| Massenbilanz | Einsatz | 8566 g | Produkte | Feststoff | 2715 g |
| | | | | Wasser | 4800 g |
| | | | | Amin | 664 g |
| | Verluste : | 351 g | | | |

Der Füllgrad des Kneters wurde im Endstadium der Trocknung bei einer Umdrehungsgeschwindigkeit von 30 U/min mit 45 % gemessen. Der gewonnene Feststoffhatte eine breite Korngrößenverteilung im Bereich 0-5 mm und eine geringe Staubneigung.

| Analytische Zusammensetzung: | | |
|---|---|---|
| Feststoff | Calcium | 40 % |
| | Sulfat | 39 % |
| | Glührückstand | 92 % (Rest: Hydratwasser) |
| | Amin: | nicht nachweisbar |
| Aminfraktion 0 -900 ml: 82 % DMEA 5 % DMIPA 8 % TEA 4,6 % Wasser DMEA = Dimethylethylamin; DMIPA = Dimethylisopropylamin; TEA = Triethylamin | | |

Wasserfraktion 1400-4800 ml: TOC 1600 mg/l (TOC = Total organic carbon/Gehalt an organischem Kohlenstoff): bedingt durch feinste Kohlenstoffpartikel.

Die Wasserfraktion mit TOC 1600 mg/l ist kläranlagenfähiges Abwasser. Dieses Wasser kann auch zum Ansetzen der Waschlösung im Aminwäscher eingesetzt werden.
Die aminreichere Wasserfraktion (0-1400 ml; ab Siedetemperatur 100 °C) wird einem weiteren Reaktionsansatz zwecks besserer Aminausbeute zugesetzt oder wird in den Aminwäscher zum Ausgleich für verdunstetes Wasser zurückgeführt. Anstelle von gelöschtem Kalk wäre in Beispiel 1 auch der Zusatz anderer zum Neutralisieren geeigneter Kalklieferformen wie Kalkmilch oder gebrannter Kalk möglich.

Da keine Rektifikation mit dem Ziel, möglichst reine Fraktionen zu gewinnen, durchgeführt wurde, sondern, wie in üblichen Knettrocknern ausschließlich möglich, eine einfache Verdampfung, lag der Wassergehalt der Aminfraktion höher als der Lage der Amin/Wasser-Azeotrope entspricht. Der Wassergehalt kann aber durch eine direkte Kopplung eines Knetverdampfers (15) incl. wärmeisoliertem Dom (2) mit Staubfilter und mit einer Rektifikationskolonne weiter reduziert werden wie in Abb. 1 skizziert. In diesem Fall können in einer diskontinuierlichen Fahrweise nacheinander die drei Aminfraktionen in hoher Reinheit getrennt und nach Kondensation im Kondensator (7) in Tanks (8a-c) isoliert werden. Während bei der Dimethylisopropylamin- und Triethylamin-Fraktion nachträglich noch eine Entwässerung erforderlich ist wegen des Wassergehalts von 4 % bzw. 10 % der Azeotrope, ist der Wassergehalt der Dimethylethylamin-Fraktion über die Bodenzahl der Kolonne (14) und das Verhältnis von Rücklaufstrom (5) und in die Tanks (8) geleiteter Produktmenge auf unter 0,3 % und damit spezifikationsgerecht einstellbar. Der sich in der Kolonne sammelnde Sumpf (16) wird über ein Regelventil (21) bevorzugt in den Knettrockner zurückgeführt. Für den Fachmann ist diese beschriebene Trennung der Amine eine einfache Trennaufgabe.

Die Kopplung von Knetverdampfer und Rektifizierkolonne stellt eine einfach realisierbare Vorrichtung dar, die aus an sich bekannten Bauteilen zusammengestellt wird. Da diese Kombination neu ist und auch auf zahlreiche andere Trennaufgaben, bei denen eine Suspension eines Feststoffs in einem Flüssigkeitsgemisch in den Feststoff und die einzelnen Bestandteile bzw. die Siedefraktionen des Flüssigkeitsgemischs getrennt werden sollen, anwendbar ist, wurde diese spezielle Vorrichtung in Anspruch 10 auch als allgemein nutzbar für Trennungen von Schlämmen in Feststoffe und mehrere Siedefraktionen beschrieben. Üblicherweise werden derartige Suspensionen getrennt, indem z. B. durch Knetverdampfung oder Filtration zuerst eine Trennung in den Feststoff und das Flüssigkeitsgemisch durchgeführt wird und anschließend z.B. in einer üblichen Rektifikationskolonne das Flüssigkeitsgemisch zerlegt wird. Insbesondere bei zähen Schlämmen mit einem Feststoffanteil >20 % kann die vorgeschlagene Vorrichtung besondere Verfahrensvorteile ermöglichen, da in einer Anlage mehrere Ziele erreicht werden konnen. Beispiele dafür sind die Auftrennung von Lackschlämmen in einen Feststoff und mehrere Fraktionen der Flüssigphase und die im folgenden Beispiel 2 beschriebene Amintrocknung.

Im folgenden Beispiel 2 wird ein einfaches Trocknungsverfahren für die wasserhaltige Aminfraktion mit Calciumoxid beschrieben; das dabei gebildete Calciumhydroxid kann danach als Base bei der Neutralisation gemäß Beispiel 1 eingesetzt werden und führt damit zu keinem Entsorgungsmehraufwand und trägt zur Schonung der Ressourcen bei.

### Beispiel 2

100 g der in Beispiel 1 gewonnenen Aminfraktion mit 4,6 % Wasser wurden in einem verschließbaren Glaskolben mit 30 g Calciumoxidpulver vermischt und über Nacht stehen gelassen. Die überstehende Aminmischung wurde danach erneut untersucht. Der Wassergehalt war auf 0,3 % gesunken. Calciumoxid hat also eine für praktische Anwendungen ausreichende Trockenwirkung auf die Amin/Wasser-Azeotrope.

Das getrocknete Amingemisch kann in einer diskontinuierlichen oder in 2 kontinierlichen Rektifikationen gemäß dem Stand der Technik getrennt werden. Es ist auch die Anwendung anderer Trockenmittel wie Zeolithen (Siebweite 4-10 Ångström) oder Alkalihydroxid möglich, jedoch ist gebrannter Kalk die bevorzugte Variante.

Die Kopplung von Knetverdampfer und Rektifizierkolonne gemäß Anspruch 10 kann dazu genutzt werden, in einer Stufe bei diskontinuierlicher Fahrweise Dimethylethylamin als am niedrigsten siedende Fraktion (Siedepunkt 36 C bei Normaldruck) in 99 % iger Reinheit und mit max. 0,3 % Wasser als verkaufsfähiges Produkt zu gewinnen, das z.B in Tank (8c) zwischengelagert wird. Die danach gewonnenen Amin/Wasser-Azeotrope werden z.B. in Tanks (8a,b) getrennt gesammelt und anschließend ,wie in Beispiel 2 beschrieben, mit gebranntem Kalk getrocknet und durch Rektifikation rein dargestellt. Zur Trocknung und Rektifikation der Azeotrope werden diese vorzugsweise über die Verbindungsleitung (20) in den Knettrockner zur chemischen Trocknung mit gebranntem Kalk (12) dosiert und nach dem Wasserentzug mit der angeschlossenen Rektifizierkolonne (14) rein dargestellt. Dieses Vorgehen ist ein weiteres Beispiel für die vielseitig nutzbare Kombination von Knettrockner und Rektifizierkolonne gemäß Anspruch 10. In diesem Fall wird der deaminierte Kalkrückstand nicht ausgeschleust, sondern bleibt als Basenvorrat im Knettrockner zwecks Neutralisation der Aminsulfatlösung in der Deaminierungsstufe.

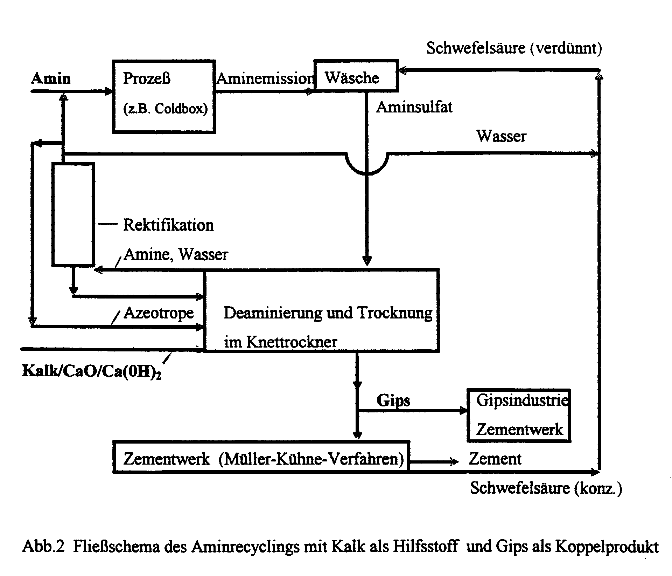

Die Vorteile der oben beschriebenen Verfahrenskombination
1.Trennung in Feststoff, Wasserphase und Amin/Wasser-Phase im Knettrockner.
2. Trocknung der Amin/Wasserphase über Calciumoxid
3. Rektifikation der getrockneten Aminphase
sind gegenüber dem bisherigen Stand der Technik:
- Vermeidung von voluminösen Destillationsrückständen
- Vermeidung von Wasserzugabe bei der Neutralisation, d.h. keine weitere Verdünnung des Aminsulfats, da Konzentrationsniederschläge nicht mehr vermieden werden müssen.
- Das Neutralisationsmittel - z.B. gebrannter Kalk oder wasserfreies Kaliumhydroxid - wird zusätzlich in seiner Eigenschaft als Trocknenhilfsmittel genutzt.
- Der Rückstand (Calciumsulfat und gelöschter Kalk) der oben beschriebenen Destillation im Knettrockner eignet sich ohne vorhergehende Neutralisation zum Einsatz als Zementrohstoff oder als Einsatzmaterial für die Gipsindustrie, z.B. in Form von Alpha-Halbhydrat.
- In einer besonderen Variante bietet sich an, den Destillationsrückstand, der zu bis zu 90 oder 95 % aus Gips besteht, im Müller-Kühne-Verfahren zu Zement und Schwefelsäure umzusetzen. Damit ist auch für die Schwefelsäurekomponente die Stoffkreislaufführung gewährleistet. Das Fließschema Abb.2 zeigt für diesen Fall die Kreislaufstromführung für Amin und Schwefelsäure auf, und der Hilfsstoff Kalk wird über den Umweg der Sulfatbindung in Form von Gips der Zementherstellung zugeführt.

Die übliche und bevorzugte Fahrweise ist die diskontinuierliche Fahrweise, wie oben beschrieben. Das Volumen des Knettrockners liegt bevorzugt bei 4 - 12 m3, aber dieser Bereich stellt keine technische Einengung dar.

Anstelle des Chargenbetriebs kann auch eine kontinuierliche Fahrweise durchgeführt werden. In diesem Fall ist eine getrennte Sammlung der einzelnen wasserarmen Aminfraktionen nicht möglich, es sei denn es werden mehrere Kolonnen und Kneter hintereinander geschaltet. Bei großen Mengen anfallender Waschlösungen kann die kontinuierliche Trocknung in entweder einem Knettrockner mit großem Länge/Durchmesser-Verhältnis (>5) und mehreren durch Stauwehre (9) voneinander abgetrennten Zonen und axialen Temperaturgradienten oder mit mehreren hintereinandergeschalteten Knettrocknern durchgeführt werden. Die Wahl zwischen kontinuierlicher und diskontinuierlicher Betriebsweise ist eine betriebswirtschaftliche Optimierungsaufgabe.

Eine vorteilhafte Variante des diskontinuierlichem Betriebs ist der semikontinuierliche Betrieb, bei dem taktweise in bevorzugt 2 hintereinandergeschalteten Knettrocknern destilliert wird. In dem ersten Trockner werden beispielsweise die Aminfraktionen bis zum Siedepunkt des Wassers abdestilliert. Anschließend wird der Dcstillationssumpf in den 2. Trockner gepumpt, in dem dann die gesamte Wasserfraktion abgetrennt wird. Bei dem 2. Trockner kann auf die Rektifizierkolonne verzichtet werden. Mit dieser Fahrweise können Energiekosten eingespart werden. Die Brüden oder Kondensate des 2. Trockners können zur Wärmeenergieversorgung des ersten Trockners genutzt werden.

Der Betriebsdruck bei der Trocknung liegt bevorzugt im Bereich 0,1-4 bar. In der Endphase der Trocknung kann die Entwässerung durch Anlegen eines Vakuums unterstützt werden. Wenn dagegen Wert auf die Bildung eines Alpha-Halbhydrats im Rückstand gelegt wird, wird ein Überdrück gewählt. In der Anfangsphase der Umsetzung ist zur Verbesserung der Kondensation und zur besseren Beherrschung der Reaktionswärme ein leichter Überdruck bis ca. 2 bar sinnvoll.

## Patentansprüche

1. Verfahren zur Rückgewinnung von Aminen und Amingemischen aus sauren Aminwaschlösungen durch Mischung und Umsetzung mit Basen wie Alkali- und Erdalkalihydroxiden und destillative Trennung in einen feststoffartigen Destillationssumpf und eine flüchtige, kondensierbare Amin/Wasserphase,
**dadurch gekennzeichnet,** daß man das Reaktionsgemisch aus Aminsalzlösung und Base in einem Knettrockner (= Knetverdampfer) mischt, umsetzt, destillativ trennt und trocknet, wobei die Basenmenge in einem stöchiometrischen Überschuß bezogen auf die Menge des Anions des Aminsalzes eingesetzt wird.

2. Verfahren nach Anspruch 1, dadurch gekennzeichnet, daß ein Druck im Bereich 0,1 - 5 bar eingestellt wird.

3. Verfahren nach Anspruch 1 oder 2, dadurch gekennzeichnet, daß man diskontinuierlich umsetzt und trocknet in 1-3 hintereinandergeschalteten Trocknern

4. Verfahren nach Anspruch 3, dadurch gekennzeichnet, daß man Aminsalz und Base bei Temperaturen unterhalb des Siedepunkts des am niedrigsten siedenden Amins miteinander vermischt und das Reaktionsgemisch zur Trocknung so erhitzt, daß die Amine, das Wasser und ggf. flüchtige organische Begleitstoffe nacheinander destillativ abgetrennt werden , wobei man als abgetrennte flüchtige Fraktionen mindestens eine wasserarme Aminfraktion und eine annähernd aminfreie Wasserphase erhält.

5. Verfahren nach einem der Ansprüche 1 - 4 , dadurch gekennzeichnet, daß man die wasserarme Aminfraktion mit einem Alkalihydroxid oder Calciumoxid trocknet und anschließend in einer Rektifizierkolonne in die reinen Komponenten zerlegt.

6. Verfahren nach einem der Ansprüche 1-5, dadurch gekennzeichnet, daß man die Trocknung so weit durchführt, daß der feste Rückstand in Form eines rieselfähigen Produkts mit <20 % Wassergehalt vorliegt.

7. Verfahren nach einem der Ansprüche 1-6, dadurch gekennzeichnet. daß man als Base Calciumoxid oder Calciumhydroxid einsetzt.

8. Verfahren nach Anspruch 7, dadurch gekennzeichnet, daß man als saure Aminwaschlösung eine Aminsulfatlösung einsetzt.

9. Verfahren nach einem der Ansprüche 1-6, dadurch gekennzeichnet, daß man als Base Kaliumhydroxid einsetzt.

10. Vorrichtung zur Durchführung des Verfahrens nach einem der Ansprüche 1-9 und zur thermischen Trennung von sonstigen Schlämmen in einen Feststoff und mehrere Flüssigfraktionen, dadurch gekennzeichnet, daß der Knettrockner indirekt beheizt ist und die Knetspaltweiten 1,5 - 10 mm betragen und daß er einen Dom besitzt, über den er mit einer Rektifizierkolonne und mit einem daran anschließenden Kondensator gekoppelt ist.

11. Vorrichtung nach Anspruch 9, dadurch gekennzeichnet, daß der Trocknerdom einen Feinstaubfilter und die Rektifizierkolonne 2-10 theoretische Böden besitzt und eine Teilrückführung des kondensierten Stroms erlaubt.

12. Vorrichtung nach einem der Ansprüche 10 und 11, dadurch gekennzeichnet, daß zur kontinuierlichen Durchführung des Verfahrens 2-3 dieser Vorrichtungen hintereinander geschaltet sind, wobei in der ersten Stufe eine Teiltrocknung und in der letzten Stufe die Endtrocknung erfolgt.

13. Verwendung des nach Anspruch 8 erhaltenen festen, aus Calciumsulfat und Calciumhydroxid bestehenden Rückstands nach Zusatz weiterer Komponenten zur Herstellung von Zement, wobei die bei der Brennung entstehenden Schwefeloxide abgetrennt und in bekannter Weise aufgearbeitet werden.

14. Verwendung des nach Anspruch 8 erhaltenen festen, aus Calciumsulfat und Calciumhydroxid bestehenden Rückstands als Komponente für Gipsprodukte.

15. Verwendung des nach Anspruch 9 erhaltenen Rückstands nach Neutralisation als Düngemittelkomponente.

## Claims

1. A process for recovering amines and amine mixtures from acid amine scrub liquors by mixture and reaction with bases as alkali metal hydroxides and alkaline earth metal hydroxides and separation by distillation into a solid-like distillation residue and a volatile, condensable amine/water phase, **comprising the steps of** mixing, reacting, separating by distillation and drying the reaction mixture of amine salt solution and base in a kneading drier (=kneading evaporator), whereby the quantity of base is used in a stoichiometric surplus corresponding to the quantity of the anion of the amine salt.

2. A process as claimed in Claim 1, wherein a pressure is adjusted in the range of 0,1-5 bar.

3. A process as claimed in Claim 1 or 2, wherein a discontinuous reaction and drying is carried through in 1-3 driers connected in series.

4. A process as claimed in Claim 3, wherein amine salt and base are mixed with each other at temperatures below the boiling point of the amine having the lowest boiling point and heating the reaction mixture for drying in a way that the amines, the water and eventually volatile organic contaminants are separated by distillation one after another, whereby obtaining at least one low-water-content amine fraction and one nearly amine-free water phase as separated volatile fractions.

5. A process as claimed in any of the Claims 1 to 4, wherein the amine fraction containing a low quantity of water is dried with an alkali hydroxide or calcium oxide and then separated in a rectification column into the pure components.

6. A process as claimed in any of the Claims 1 to 5, wherein the drying is carried through to an extent that the solid residue is present in form of a pourable product with < 20% water content.

7. A process as claimed in any of the Claims 1 to 6, wherein calcium oxide or calcium hydroxide is used as base.

8. A process as claimed in Claim 7, wherein an amine sulphate solution is used as acid amine scrub liquor.

9. A process as claimed in any of the Claims 1 to 6, wherein potassium hydroxide is used as base.

10. A device for carrying through the process as claimed in any of the Claims I to 9, and for thermic separation of other sludges into a solid and several liquid fractions, wherein the kneading drier is heated indirectly and the width of the kneading gaps is 1.5 - 10 mm and is equipped with a dome through which it is coupled with a rectification column and with a condenser connected to that.

11. A device as claimed in Claim 9, wherein the drying dome has a fine dust filter and the rectification column has 2-10 theoretical plates and a partial feedback of the condensed flow is possible.

12. A device as claimed in any of the Claims 10 and 11, wherein 2-3 of these devices are connected in series for the continuous execution of the process, whereby a partial drying takes place in the first stages and the final drying takes place in the last stage.

13. A use of the residue of calcium sulphate and calcium hydroxide, obtained as claimed in claim 8, after the addition of further components for the production of cement, whereby sulphur oxides developing during calcination are separated and processed in the well-known manner.

14. A use of the solid residue consisting of calcium sulphate and calcium hydroxide obtained as claimed in claim 8 as component part for gypsum products.

15. A use of the residue obtained as claimed in claim 9 as fertiliser component after neutralisation.

## Revendications

1. Procédé permettant de récupérer les amines et les mélanges d'amines à partir de solutions de lavage acides par mélange et réaction avec des bases comme les hydroxydes alcalins et alcalino-terreux, et séparation par distillation en un résidu de distillation solide et une phase d'amine et d'eau condensable,
**caractérisé** par le fait que l'on mélange, transforme, sépare par distillation et déshydrate le mélange réactionnel composé d'une solution saline aminée et d'une base dans un dessiccateur malaxeur ( = évaporateur malaxeur), la quantité de base utilisée présentant un excès stoechiométrique par rapport à la quantité de l'anion du sel aminé.

2. Procédé conforme à la revendication n° 1, caractérisé par le fait que la pression est réglée entre 0,1 et 5 bars.

3. Procédé conforme à la revendication n° 1 ou 2, caractérisé par le fait que la réaction est discontinue et que la déshydratation a lieu dans 1 à 3 dessiccateurs montés en série.

4. Procédé conforme à la revendication n° 3, caractérisé par le fait que le sel aminé et la base sont mélangés à une température inférieure au point d'ébullition de l'amine possédant la température d'ébullition la plus basse et que le mélange réactionnel est chauffé pour la déshydratation de façon à pouvoir séparer successivement l'eau et le cas échéant, les impuretés organiques volatiles, par distillation. Les fractions volatiles séparées obtenues se composent alors au moins d'une fraction d'amine de faible teneur en eau et d'une phase d'eau ne contenant pratiquement pas d'amines.

5. Procédé conforme à l'une des revendications 1 à 4, caractérisé par le fait que la fraction d'amines pauvre en eau est déshydratée au moyen d'un hydroxyde alcalin ou d'oxyde de calcium, puis décomposée en ses composants purs dans une colonne de rectification.

6. Procédé conforme à l'une des revendications 1 à 5, caractérisé par le fait que l'on poursuit la déshydratation jusqu'à ce que le résidu solide se présente sous forme produit de bonne faculté d'écoulement, contenant moins de 20 % d'eau.

7. Procédé conforme à l'une des revendications 1 à 6, caractérisé par le fait que l'on utilise de l'oxyde ou de l'hydroxyde de calcium comme base.

8. Procédé conforme à la revendication 7, caractérisé par le fait que l'on utilise une solution de sulfate aminée comme solution acide de lavage des amines.

9. Procédé conforme à l'une des revendication 1 à 6, caractérisé par le fait que l'on utilise de l'hydroxyde de potassium comme base.

10. Dispositif permettant de réaliser le procédé selon l'une des revendications 1 à 9 et de séparer thermiquement d'autres boues en une matière solide et plusieurs fractions liquides, caractérisé par le fait que le dessiccateur malaxeur est indirectement chauffé et que l'espace entre les éléments de malaxage est compris entre 1,5 et 10 mm, et qu'il est doté d'un dôme par lequel il est couplé à une colonne de rectification, elle-même raccordée à un condenseur.

11. Dispositif conforme à la revendication 9, caractérisé par le fait que le dôme du dessiccateur possède un filtre à poussières fin et que la colonne de rectification renferme 2 à 10 plateaux théoriques et permet de reconduire partiellement le flux condensé.

12. Dispositif conforme à l'une des revendications 1 à 11, caractérisé par le fait que 2 à 3 de ces dispositifs sont montés en série pour pouvoir réaliser le procédé en continu, une déshydratation partielle ayant lieu pendant la première phase et la déshydratation finale pendant la deuxième phase.

13. Utilisation du résidu solide composé de sulfate et d'hydroxyde de calcium, obtenu dans le procédé décrit dans la revendication 8, après addition d'autres composants pour fabriquer du ciment, les oxydes de calcium résultant de la combustion étant séparés et traités selon la méthode habituelle.

14. Utilisation du résidu solide composé de sulfate et d'hydroxyde de calcium, obtenu dans le procédé décrit dans la revendication 8, comme composant de produits gypseux.

15. Utilisation du résidu obtenu dans le procédé décrit dans la revendication 9, comme composant d'engrais après neutralisation.
